# EUROPÄISCHE PATENTSCHRIFT

(11) **EP 3 513 187 B1**
(45) Veröffentlichungstag und Bekanntmachung des Hinweises auf die Patenterteilung: **07.08.2024**
(21) Anmeldenummer: 17768436.2
(22) Anmeldetag: 14.09.2017
(51) Int. Cl.: G01N 33/50, B01L 3/00, G02B 21/00

(54) **VERFAHREN ZUM DURCHFÜHREN EINES ALLERGIETESTS, VERFAHREN ZUM BESTIMMEN EINER DEGRANULATION BEI ZELLEN UND VORRICHTUNG ZUM DURCHFÜHREN EINES ALLERGIETESTES**
METHOD FOR CARRYING OUT AN ALLERGY TEST, METHOD FOR DETERMINING DEGRANULATION IN CELLS AND DEVICE FOR CARRYING OUT AN ALLERGY TEST
PROCÉDÉ PERMETTANT D'EFFECTUER UN TEST D'ALLERGIE, PROCÉDÉ PERMETTANT DE DÉTERMINER UNE DÉGRANULATION DE CELLULES ET DISPOSITIF SERVANT À EFFECTUER UN TEST D'ALLERGIE

(30) Priorität: 15.09.2016 DE 102016117421
(43) Veröffentlichungstag der Anmeldung: 24.07.2019
(73) Patentinhaber: Universität Bremen, 28359 Bremen (DE); Medizinische Universität Wien, 1090 Wien (AT)
(72) Erfinder: VAN DEN DRIESCHE, Sander, 28359 Bremen (DE); VELLEKOOP, Michael, 28359 Bremen (DE); FALLDORF, Claas, 28205 Bremen (DE); HAFNER, Christine, 1040 Wien (AT); BREITENEDER, Heimo, 1070 Wien (AT)
(74) Vertreter: Tappe, Udo
(86) Internationale Anmeldenummer: PCT/EP2017/073144
(87) Internationale Veröffentlichungsnummer: WO 2018/050749

(56) Entgegenhaltungen:
- WO-A1-2013/083815
- WO-A1-2013/083815
- WO-A1-93/25904
- WO-A1-93/25904
- WO-A1-93/25904
- DE-A1- 102014 200 911
- DE-A1- 102014 200 911
- DE-A1- 102014 200 911
- DE-A1- 102014 205 535
- DE-A1- 102014 205 535
- US-A1- 2016 131 882
- US-A1- 2016 131 882
- R HASTIE: "The antigen-induced degranulation of basophil leucocytes from atopic subjects, studied by phase-contrast microscopy", CLINICAL AND EXPERIMENTAL IMMUNOLOGY, 1 January 1971 (1971-01-01), pages 45 - 61, XP055429915, Retrieved from the Internet <URL:https://www.ncbi.nlm.nih.gov/pmc/articles/PMC1712904/pdf/clinexpimmunol00374-0053.pdf>
- L A S CARMO ET AL: "CD63 is tightly associated with intracellular, secretory events chaperoning piecemeal degranulation and compound exocytosis in human eosinophils", JOURNAL OF LEUKOCYTE BIOLOGY, vol. 100, no. 2, 10 March 2016 (2016-03-10), pages 391 - 401, XP055428147, DOI: 10.1189/jlb.3A1015-480R
- M KUROSAWA ET AL: "Phase-contrast microscopic studies using cinematographic techniques and scanning electron microscopy on IgE-mediated degranulation of cultured human mast cells", CLINICAL & EXPERIMENTAL ALLERGY, vol. 28, no. 8, 1 August 1998 (1998-08-01), pages 1007 - 1012, XP055429964, DOI: 10.1046/j.1365-2222.1998.00352.x
- R SHER ET AL: "Eosinophil degranulation : Monitoring by interference contrast microscopy", INFLAMMATION, vol. 5, no. 1, 1 March 1981 (1981-03-01), pages 37 - 53, XP055428794, DOI: 10.1007/BF00910778
- H BOCANEGRA EVANS ET AL: "Holographic microscopy and microfluidics platform for measuring wall stress and 3D flow over surfaces textured by micro-pillars", SCIENTIFIC REPORTS, vol. 6, no. 1, 29 June 2016 (2016-06-29), XP055755512, DOI: 10.1038/srep28753
- ZOLTAN GOROCS ET AL: "On-Chip Biomedical Imaging", IEEE REVIEWS IN BIOMEDICAL ENGINEERING, IEEE, USA, vol. 6, 1 January 2013 (2013-01-01), pages 29 - 46, XP011499449, ISSN: 1937-3333, DOI: 10.1109/RBME.2012.2215847
- KUO-WEI HUANG ET AL: "Optoelectronic tweezers integrated with lensfree holographic microscopy for wide-field interactive cell and particle manipulation on a chip", LAB ON A CHIP, vol. 13, no. 12, 1 January 2013 (2013-01-01), pages 2278, XP055250778, DOI: 10.1039/c3lc50168j
- M HAAPALAINEN ET AL: "Characterizing electrokinetic mobility of microparticles using in-line holographic microscopy", PHOTONICS LETTERS OF POLAND, vol. 3, no. 2, 30 June 2011 (2011-06-30), XP055756060, DOI: 10.4302/plp.2011.2.14
- "BioMEMS and Biomedical Nanotechnology", 1 January 2006, SPRINGER US, Boston, MA, ISBN: 978-0-38-725566-8, article J VOLDMAN: "Dielectrophoretic Traps for Cell Manipulation", pages: 159 - 186, XP055040051, DOI: 10.1007/978-0-387-25845-4_8
- SHU-HSIEN LIAO ET AL: "A capillary dielectrophoretic chip for real-time blood cell separation from a drop of whole blood", BIOMICROFLUIDICS, vol. 7, no. 2, 1 March 2013 (2013-03-01), pages 024110, XP055755804, DOI: 10.1063/1.4802269
- C DEPEURSINGE ET AL: "Cell Biology Explored with Digital Holographic Microscopy", BIOMEDICAL OPTICS, vol. 6880110, 1 January 2008 (2008-01-01), pages BMD58, XP055428344, DOI: 10.1364/BIOMED.2008.BMD58
- R HASTIE: "The antigen-induced degranulation of basophil leucocytes from atopic subjects, studied by phase-contrast microscopy", CLINICAL AND EXPERIMENTAL IMMUNOLOGY, 1 January 1971 (1971-01-01), pages 45 - 61, XP055429915, Retrieved from the Internet <URL:https://www.ncbi.nlm.nih.gov/pmc/articles/PMC1712904/pdf/clinexpimmunol00374-0053.pdf>
- L A S CARMO ET AL: "CD63 is tightly associated with intracellular, secretory events chaperoning piecemeal degranulation and compound exocytosis in human eosinophils", JOURNAL OF LEUKOCYTE BIOLOGY, vol. 100, no. 2, 10 March 2016 (2016-03-10), pages 391 - 401, XP055428147, DOI: 10.1189/jlb.3A1015-480R
- M KUROSAWA ET AL: "Phase-contrast microscopic studies using cinematographic techniques and scanning electron microscopy on IgE-mediated degranulation of cultured human mast cells", CLINICAL & EXPERIMENTAL ALLERGY, vol. 28, no. 8, 1 August 1998 (1998-08-01), pages 1007 - 1012, XP055429964, DOI: 10.1046/j.1365-2222.1998.00352.x
- R SHER ET AL: "Eosinophil degranulation : Monitoring by interference contrast microscopy", INFLAMMATION, vol. 5, no. 1, 1 March 1981 (1981-03-01), pages 37 - 53, XP055428794, DOI: 10.1007/BF00910778
- H BOCANEGRA EVANS ET AL: "Holographic microscopy and microfluidics platform for measuring wall stress and 3D flow over surfaces textured by micro-pillars", SCIENTIFIC REPORTS, vol. 6, no. 1, 29 June 2016 (2016-06-29), XP055755512, DOI: 10.1038/srep28753
- ZOLTAN GOROCS ET AL: "On-Chip Biomedical Imaging", IEEE REVIEWS IN BIOMEDICAL ENGINEERING, IEEE, USA, vol. 6, 1 January 2013 (2013-01-01), pages 29 - 46, XP011499449, ISSN: 1937-3333, DOI: 10.1109/RBME.2012.2215847
- KUO-WEI HUANG ET AL: "Optoelectronic tweezers integrated with lensfree holographic microscopy for wide-field interactive cell and particle manipulation on a chip", LAB ON A CHIP, vol. 13, no. 12, 1 January 2013 (2013-01-01), pages 2278, XP055250778, DOI: 10.1039/c3lc50168j
- M HAAPALAINEN ET AL: "Characterizing electrokinetic mobility of microparticles using in-line holographic microscopy", PHOTONICS LETTERS OF POLAND, vol. 3, no. 2, 30 June 2011 (2011-06-30), XP055756060, DOI: 10.4302/plp.2011.2.14
- "BioMEMS and Biomedical Nanotechnology", 1 January 2006, SPRINGER US, Boston, MA, ISBN: 978-0-38-725566-8, article J VOLDMAN: "Dielectrophoretic Traps for Cell Manipulation", pages: 159 - 186, XP055040051, DOI: 10.1007/978-0-387-25845-4_8
- SHU-HSIEN LIAO ET AL: "A capillary dielectrophoretic chip for real-time blood cell separation from a drop of whole blood", BIOMICROFLUIDICS, vol. 7, no. 2, 1 March 2013 (2013-03-01), pages 024110, XP055755804, DOI: 10.1063/1.4802269
- C DEPEURSINGE ET AL: "Cell Biology Explored with Digital Holographic Microscopy", BIOMEDICAL OPTICS, vol. 6880110, 1 January 2008 (2008-01-01), pages BMD58, XP055428344, DOI: 10.1364/BIOMED.2008.BMD58
- R HASTIE: "The antigen-induced degranulation of basophil leucocytes from atopic subjects, studied by phase-contrast microscopy", CLINICAL AND EXPERIMENTAL IMMUNOLOGY, vol. 8, 1 January 1971 (1971-01-01), pages 45 - 61, XP055429915, Retrieved from the Internet <URL:https://www.ncbi.nlm.nih.gov/pmc/articles/PMC1712904/pdf/clinexpimmunol00374-0053.pdf>
- L. A. S. CARMO ET AL: "CD63 is tightly associated with intracellular, secretory events chaperoning piecemeal degranulation and compound exocytosis in human eosinophils", JOURNAL OF LEUKOCYTE BIOLOGY, vol. 100, no. 2, 10 March 2016 (2016-03-10), US, pages 391 - 401, XP055428147, ISSN: 0741-5400, DOI: 10.1189/jlb.3A1015-480R
- M KUROSAWA ET AL: "Phase-contrast microscopic studies using cinematographic techniques and scanning electron microscopy on IgE-mediated degranulation of cultured human mast cells", CLINICAL & EXPERIMENTAL ALLERGY, vol. 28, no. 8, 1 August 1998 (1998-08-01), pages 1007 - 1012, XP055429964
- R. SHER ET AL: "Eosinophil degranulation : Monitoring by interference contrast microscopy", INFLAMMATION., vol. 5, no. 1, 1 March 1981 (1981-03-01), US, pages 37 - 53, XP055428794, ISSN: 0360-3997, DOI: 10.1007/BF00910778

## Beschreibung

Die Erfindung betrifft ein Verfahren zum Durchführen eines Allergietests, bei dem eine Blutprobe mit mindestens einem Allergen in vitro kontaktiert wird, und bei dem mindestens eine allergische Reaktion oder ein Ausbleiben der mindestens einen allergischen Reaktion von Immunzellen mittels einer Mikroskopiereinrichtung direkt optisch beobachtet wird. Des Weiteren betrifft die Erfindung ein Verfahren zum Bestimmen einer Degranulation bei Immunzellen, bei dem eine Blutprobe mit mindestens einem Reaktionspartner in vitro kontaktiert wird. Darüber hinaus betrifft die Erfindung eine Vorrichtung zum Durchführen eines Allergietests mit einem Verfahren der eingangs genannten Art, mit einer Mikroskopiereinrichtung und mit einem mindestens teilweise transparenten mikrofluidischen Chip zum direkten optischen Beobachten einer allergischen Reaktion oder zum Beobachten des Ausbleibens einer allergischen Reaktion.

Ein Verfahren zum Durchführen eines Allergietests ist in dem Dokument R. Hastie "The antigen-induced degranulation of basophil leucocytes from atopic subjects, studied by phase-contrast microscopy", Clinical and Experimental Immunology, 8, 1971, 45-61 beschrieben. Die Druckschrift DE 10 2014 200 911 A1 zeigt ein Verfahren zum Aufnehmen des Höhenverlaufes oder der Höhenkontur einer Zelle. Die Dokumente L. CARMO ET AL, J. LEUKOCYTE BIOL., 100(2), 2016, 391-401 und R. SHER ET AL, INFLAMMATION, 5(1), 1981, 37-53 zeigen Verfahren zur Bestimmung einer Degranulation bei Eosinophilen. Das Dokument M. KUROSAWA ET AL, CLIN. EXP. ALLERGY, 28(8), 1998, 1007-1012 zeigt ein Verfahren zur Bestimmung einer Degranulation bei Mastzellen. Die Druckschrift WO 93/25904 A1 zeigt ein alternatives Verfahren zum Durchführen eines Allergietests bei dem die Zahl der freigesetzten Granula mittels eines Streuungsdiagramms bestimmt wird. Darüber hinaus offenbaren H. BOCANEGRA EVANS ET AL, SCIENTIFIC REPORTS, 6(1), 2016, Z. GOROCS ET AL, IEEE REVIEWS IN BIOMEDICAL ENGINEERING, 6, 2013, 29-46, DE 10 2014 205535 A1, US 2016/131882 A1, KUO-WEI HUANG ET AL, LAB ON A CHIP, 13(12), 2013, 2278, M. HAAPALAINEN ET AL, PHOTONICS LETTERS OF POLAND, 3(2), 2011, 85-87, WO 2013/083815 A1, und C. DEPEURSINGE ET AL, BIOMEDICAL OPTICS, 6880110, 2008, BMD58 holographische Mikroskope.

Im Rahmen der vorliegenden Anmeldung wird der Begriff Blutprobe synonym verwendet für eine direkte Blutprobe oder ein Serum aus einer direkten Blutprobe, das mit Zellen von speziellen Zelllinien versetzt oder in Kontakt gebracht wurde. Die speziellen Zelllinien können vor allem humanisierte, insbesondere Rattenzellen und/oder Meerschweinchenzellen sein.

Entsprechende Verfahren zum Durchführen eines Allergietests sind als Bluttests bekannt, bei denen ein Immunglobolin-Antikörper-Spiegel, insbesondere ein Immunglobolin-E-Spiegel, bestimmt wird. Zellen der körpereigenen Immunabwehr enthalten Granula mit beispielsweise Histamin, das sie bei Aktivierung ausschütten. Insbesondere erfolgt ein Aktivierungsprozess über das Immunglobolin E (IgE), welches bei einer Sensibilisierung an die Zellmembran einer Immunzelle gebunden ist. Bei einer vorhandenen Sensibilisierung für eine bestimmtes Allergen kann ein Degranulationsprozess veranlasst werden, bei dem Granula aus der Immunzelle ausgeschüttet werden. Hierbei stellt die Degranulation bzw. das Ausschütten der Granula aus der Immunzelle eine Initiierung einer allergischen Entzündungsreaktion dar.

Hierbei ist von Nachteil, dass die Feststellung einer Allergie auf dem antikörper-basierten Nachweis von IgE basiert. IgE ist ein Marker für eine allergische Sensibilisierung, eine Allergie, eine Entzündung und/oder bestimmte Bluterkrankungen. Dies bedeutet, dass bei einem antikörper-basierten Nachweis einer Allergie auf der Basis der Auswertung von IgE-Antikörpern nicht zwingend eine Allergie vorliegen muss, auch wenn der Test selbst positiv ist. Hierdurch besteht das Risiko einer hohen Anzahl an Fehlinterpretationen der Testergebnisse. So kann beispielsweise der Anteil von falschen positiven Interpretationen der Testergebnisse zum Feststellen einer Allergie im Bereich von 50 bis 60 % liegen. Der Anteil von falschen negativen Testergebnissen kann im Bereich von 15 bis 20 % liegen.

Um falsche Testergebnisse zu identifizieren bzw. eine Allergie zu bestätigen ist es bekannt, einen sogenannten Provokationstest durchzuführen. Beispielsweise kann ein oraler Provokationstest durchgeführt werden. Hierbei ist jedoch von Nachteil, dass dies für die jeweils getestete Person bzw. den Patienten aufgrund der provozierten allergischen Reaktion sehr unangenehm sein kann. Insbesondere besteht bei einem Provokationstest die Gefahr eines lebensbedrohlichen anaphylaktischen Schocks.

Darüber hinaus ist von Nachteil, dass es sich bei einem Allergietest auf der Basis eines antikörper-basierten Nachweises von IgE um eine indirekte Methode handelt. Insbesondere erfolgt der Nachweis der in einer Blutprobe ermittelten IgE-Moleküle mittels Enzym-markierter Antikörper gegen IgE. Ein entsprechender Komplex kann mit einer zweiten Entwicklungsreaktion, insbesondere mittels Fluoreszenz, nachgewiesen werden. Somit kann eine allergische Reaktion lediglich indirekt mittels indirekten Allergieindikatoren, insbesondere IgE-Antikörpern oder Fluoreszenz, nachgewiesen werden.

Es ist daher die der Erfindung zugrundeliegende Aufgabe, ein Verfahren und eine Vorrichtung der eingangs genannten Art derart weiterzuentwickeln, dass ein Allergietest mit einer erhöhten Aussagekraft und/oder besseren Genauigkeit durchgeführt werden kann. Insbesondere soll ein schnellerer und/oder kostengünstigerer Allergietest zur Verfügung gestellt werden. Des Weiteren soll mindestens eine alternative Ausführungsform bereitgestellt werden.

Die der Erfindung zugrundeliegende Aufgabe wird mit einem Verfahren mit den Merkmalen des Patentanspruchs 1 und mit einer Vorrichtung mit den Merkmalen des Patentanspruchs 12 gelöst.

Hierbei ist von Vorteil, dass eine allergische Reaktion unmittelbar bzw. direkt bestimmbar ist. Insbesondere ist eine Degranulation von, vorzugsweise bestimmten oder vorgegeben, Immunzellen unmittelbar beobachtbar. Hierbei kann eine Degranulation als ein relevanter Parameter zum Feststellen einer allergischen Reaktion aufgefasst werden. Aufgrund einer Visualisierung eines Degranulationsprozesses kann somit eine direkte Methode zum Feststellen einer Allergie bereitgestellt werden. Dies ermöglicht eine verbesserte und zuverlässigere Bestimmung von Allergien. Hierdurch kann auf indirekte Methoden zum Feststellen einer Allergie verzichtet werden. Zudem kann eine direkte optische Beobachtung einer allergischen Reaktion die Realisierung eines genaueren, schnelleren und/oder kostengünstigeren Allergietests ermöglichen.

Vorzugsweise ist im Rahmen der vorliegenden Anmeldung unter einem direkten optischen Beobachten ein Bestimmen, Überwachen, Detektieren und/oder Messen zu verstehen. Insbesondere ist unter einem direkten optischen Beobachten ein Aufnehmen und/oder Auswerten von Daten und/oder Bildern zu verstehen. Ein direktes optisches Beobachten kann als ein quantitatives Mikroskopieren, insbesondere ein quantitatives Phasenkontrastmikroskopieren, ausgebildet sein. Im Unterschied zu einem indirekten Bestimmen einer allergischen Reaktion mittels eines Markers oder indirekten Allergieindikators kann mittels der Erfindung eine allergische Reaktion direkt bzw. unmittelbar bestimmt, beobachtet und/oder gemessen werden. Somit kann bei einem direkten Beobachten, Bestimmen, Überwachen, Detektieren und/oder Messen einer allergischen Reaktion auf eine aufwändigere Auswertung eines Markes oder indirekten Allergieindikators verzichtet werden. Hierdurch ist eine Allergie genauer, mit höherer Zuverlässigkeit, schneller und/oder kostengünstiger realisierbar.

Bei der Erfindung wird bei einer allergischen Reaktion eine Degranulation von, insbesondere bestimmten und/oder vorgegebenen, Immunzellen der Blutprobe mit der Mikroskopiereinrichtung, insbesondere quantitativ, beobachtet. Hierbei sind die Immunzellen Bestandteile der Blutprobe. Die Blutprobe kann dabei eine direkte Blutprobe sein oder ein Serum aus einer direkten Blutprobe, das mit Zellen von speziellen Zelllinien versetzt oder in Kontakt gebracht wurde. Vorzugsweise handelt es sich bei den Immunzellen um Granulozyten, basophile Zellen, basophile Granulozyten und/oder Mastzellen. Insbesondere werden Granula von Zellen beobachtet, überwacht, vermessen und/oder aufgenommen. Insbesondere wird ein Bewegungsweg der Granula für eine vorgegebene Zeit beobachtet, vermessen und/oder aufgenommen. Vorzugsweise wird beobachtet, ob Granula einer Zelle nach einer Kontaktierung der Zelle mit dem mindestens einen Allergen innerhalb einer vorgegebenen Zeit aus der Zelle austreten bzw. ausgeschüttet werden. Vorzugsweise sind im Rahmen der vorliegenden Anmeldung unter Immunzellen derartige Zelltypen einer Blutprobe zu verstehen, die potentiell eine Sensibilisierung für eine allergische Reaktion aufweisen können. Aufgrund der direkten optischen Beobachtung der Immunzellen bei Kontaktierung der Zellen mit dem mindestens einen Allergen kann ein Ausbleiben der Degranulation als nicht-allergische Reaktion klassifiziert werden. Bei einem Stattfinden der Degranulation kann dies als eine allergische Reaktion klassifiziert werden. Somit kann aufgrund der Beobachtung der Granula-Bewegung auf direkte Art und Weise und/oder in Echtzeit festgestellt werden, ob eine allergische Sensibilisierung bzw. Allergie vorliegt oder nicht.

Gemäß einer Weiterbildung wird nach der Entnahme der Blutprobe und vor dem Kontaktieren mit dem mindestens einen Allergen die Blutprobe zum Erhöhen des Anteils an Immunzellen präpariert. Hierbei kann die Entnahme der Blutprobe in an sich bekannter Weise erfolgen. Beispielsweise wird die Blutprobe einer Person bzw. einem Patienten mittels einer Nadel oder einer Spritze entnommen. Hierbei kann eine vergleichsweise geringe Menge an Blut als Blutprobe ausreichen. Vorzugsweise ist eine Blutmenge von weniger als 100 ml, weniger als 10 ml oder weniger als 1 ml ausreichend. Ihm Rahmen der Präparation der Blutprobe kann die Blutprobe mit Zusätzen versehen werden. Hierbei kann es sich beispielsweise um Natriumcitrat und/oder um Herapin handeln. Aufgrund einer geeigneten Präparation der Blutprobe kann eine Gerinnung des Blutes vermieden oder verzögert werden. Insbesondere wird der prozentuale Anteil von Immunzellen in der Blutprobe erhöht. Beispielsweise kann der prozentuale Anteil von Immunzellen in der Blutprobe auf mehr als 5 %, mehr als 10 %, mehr als 20 % oder größer erhöht werden.

Vorzugsweise wird im Rahmen der Präparation der Blutprobe der Anteil an roten Blutzellen reduziert. Die Reduzierung oder eine Entfernung von roten Blutzellen aus der Blutprobe kann mittels üblicher, insbesondere chemischer, elektrischer und/oder mechanischer, Methoden realisiert werden. Insbesondere wird der Anteil an von den zu untersuchenden Immunzellen verschiedenen weißen Blutzellen reduziert. Der Anteil an den zu untersuchenden Immunzellen, insbesondere von Granulozyten, basophilen Zellen, basophilen Granulozyten und/oder Mastzellen, kann durch die Entfernung hiervon abweichenden weißen Blutzellen erhöht werden. Insbesondere können basophile Zellen aufgrund einer Entfernung aller anderen weißen Blutzellen isoliert werden. Hierzu können an sich bekannte und/oder übliche Verfahren, Methoden oder Techniken eingesetzt werden, Aufgrund der Präparation der Blutprobe ist eine Erhöhung des Anteils von zu untersuchenden Immunzellen in der Blutprobe auf mehr als 5 %, mehr als 10 %, mehr als 20 % oder höher erreichbar. Insbesondere ist ein Anteil in einen Bereich von 10 % bis 20 % oder höher realisierbar. Für das Bestimmen einer Degranulation der zu untersuchenden Zellen und/oder dem Beobachten einer allergischen Reaktion ist es somit nicht notwendig, die zu untersuchenden Zellen vollständig von sämtlichen anderen Bestandteilen der Blutprobe zu isolieren. Die präparierte Blutprobe kann zum Verbessern der Lagerfähigkeit, insbesondere für eine Zeit von mehreren Stunden, in ein geeignetes Medium suspendiert werden.

Bei der Vorrichtung mit den Erfindungsmerkmalen wird ein mikrofluidischer Chip zum Kontaktieren der Blutprobe mit dem mindestens einen Allergen verwendet. Mittels eines mikrofluidischen Chips kann bereits eine vergleichsweise geringe Menge der Blutprobe, insbesondere im Bereich eines Tropfens oder mehrerer Tropfen, für eine Untersuchung bereitgestellt werden. Ein mikrofluidischer Chip kann einen Mikrokanal oder mehrere Mikrokanäle aufweisen. Des Weiteren kann ein mikrofluidischer Chip aus mehreren Schichten aufgebaut sein. Eine, mindestens einen Mikrokanal und/oder mindestens einen Reaktionsraum aufweisende Schicht, kann aus Silizium gebildet sein. Eine obere und/oder untere Deckschicht oder Trägerschicht kann aus einem transparenten Material, insbesondere Glas oder Kunststoff, gebildet sein. Vorzugsweise ist eine Siliziumschicht zwischen zwei transparenten Schichten, vorzugsweise Glasschichten, angeordnet. Insbesondere werden Immunzellen der Blutprobe in mindestens einem Reaktionsraum des Chips angeordnet. Der mikrofluidische Chip kann mehrere Reaktionsräume zum Aufnehmen von Immunzellen der Blutprobe aufweisen. Hierbei können die mehreren Reaktionsräume voneinander getrennt und/oder mittels eines oder mehrerer Mikrokanäle miteinander verbunden sein. Vorzugsweise ist der Reaktionsraum und/oder mindestens ein Referenzraum des mikrofluidischen Chips mindestens teilweise transparent ausgebildet. Der mindestens eine Reaktionsraum und/oder der mindestens eine Referenzraum kann mindestens einseitig transparent ausgebildet und/oder abgedeckt sein. Insbesondere ist unter Transparenz eine Durchlässigkeit in Bezug auf elektromagnetische Wellen und/oder Licht unterschiedlicher Wellenlängen zu verstehen.

Bei der Erfindung wird mindestens ein Allergen in den mindestens einen Reaktionsraum geführt. Insbesondere nachdem mehrere Immunzellen in den mindestens einen Reaktionsraum angeordnet wurden, wird mindestens ein Allergen hinzugefügt. Hierbei kann ein vorgegebenes, einzelnes Allergen in einen bestimmten, vorgegebenen Reaktionsraum geführt werden. Alternativ können mehrere Allergene, insbesondere eine bestimmte Anzahl vorgegebener Allergentypen, in den Reaktionsraum geführt werden. Insbesondere wird das mindestens eine Allergen sowohl in den mindestens einen Reaktionsraum mit den Immunzellen als auch in einen Referenzraum ohne Immunzellen geführt. Der Referenzraum kann ebenfalls mittels der Mikroskopiereinrichtung direkt optisch beobachtet werden. Insbesondere erfolgt die Beobachtung des mindestens einen Reaktionsraumes und des zugeordneten Referenzraumes gemeinsam und/oder zeitgleich. Hierbei kann die Beobachtung des Referenzraumes ohne Immunzellen im Vergleich mit der Beobachtung des zugehörigen Reaktionsraumes mit den Immunzellen die Auswertung und/oder die Qualität der Beobachtungsergebnisse verbessern. Insbesondere wird mittels der Beobachtung des mindestens eines Reaktionsraumes und des zugeordneten Referenzraumes ein quantitativer Phasenkontrast interferometrisch gemessen. Vorzugsweise ist jedem Reaktionsraum jeweils ein Referenzraum zugeordnet. Bei einem mikrofluidischen Chip mit mehreren Reaktionsräumen ist somit eine gleiche Anzahl von Referenzräumen vorhanden. Die Immunzellen einerseits und das mindestens eine Allergen andererseits können mittels desselben Mikrokanals oder mittels unterschiedlicher Mikrokanäle in den mindestens einen Reaktionsraum und/oder in den mindestens einen Referenzraum geführt werden.

Bei der Vorrichtung mit den Erfindungsmerkmalen werden die Immunzellen der Blutprobe mittels eines geeigneten Verfahrens und/oder einer geeigneten Vorrichtung auf oder in dem mikrofluidischen Chip angeordnet oder positioniert. Dabei werden Immunzellen der Blutprobe mittels einer mikrofluidischen Zellenfalle, einer Elektrophorese, einer Dielektrophorese und/oder mechanischen Methoden auf oder in dem mikrofluidischen Chip angeordnet. Bei der Dielektrophorese kann ein inhomogenes elektrisches Feld zum Bewegen, Trennen, Anordnen und/oder Positionieren von Zellen bzw. Immunzellen genutzt werden. Aufgrund des inhomogenen elektrischen Feldes kann in den Zellen ein Dipolmoment induziert werden, das sodann in Wechselwirkung mit dem angelegten elektrischen Feld tritt. Hierbei erfahren die Zellen eine Kraft und bewegen sich, je nach Feld und Dipolmoment, in Bereiche hoher oder niedriger Feldstärke. Die Kraftwirkung kann proportional zum Volumen der Zellen sein. Somit können die Zellen, insbesondere die Immunzellen, in einer Art "Feldkäfig" eingefangen werden. Insbesondere werden Immunzellen mittels der Elektrophorese und/oder Dielektrophorese in mindestens einem Reaktionsraum positioniert und/oder gehalten. Vorzugsweise lassen sich Immunzellen der Blutprobe mittels Dielektrophorese aufteilen und in mehreren Reaktionsräumen anordnen. Aufgrund der Trennung der Immunzellen in mehrere Reaktionsräume können zeitgleich unterschiedliche Allergene mit Immunzellen in voneinander getrennten Reaktionsräumen kontaktiert werden. In Abhängigkeit von der gewählten Anzahl an Reaktionsräumen und/oder verwendeten Allergenen können somit zeitgleich mehrere allergische Sensibilisierungen bzw. Allergien getestet werden.

Vorzugsweise wird mindestens ein Referenzraum mittels der Elektrophorese und/oder Dielektrophorese ohne bzw. frei von Immunzellen bereitgestellt. Somit kann mittels der Elektrophorese und/oder Dielektrophorese ein Referenzraum zur Verfügung gestellt werden, in dem sich keine Immunzellen befinden. Die Immunzellen können innerhalb des mikrofluidischen Chips mittels der Elektrophorese und/oder Dielektrophorese derart gesteuert werden, dass der Bereich des Referenzraumes zellfrei wird und/oder bleibt. Auf diese Weise kann der Immunzellen-freie Referenzraum mit dem Immunzellen enthaltenen mindestens einen Reaktionsraum optisch überlagert werden. Hierdurch kann ein quantitativer Phasenkontrast der Immunzellen interferometrisch gemessen werden. Insbesondere überlagert sich das Licht, dass mindestens teilweise durch den Reaktionsraum und mindestens teilweise durch den Referenzraum verläuft. Das sich überlagernde Licht kann einen im Wesentlichen oder annähernd identischen Weg, insbesondere durch den mikrofluidischen Chip, aufweisen. Hierdurch kann eine Empfindlichkeit gegenüber äußeren mechanischen Einflüssen reduziert werden. Insbesondere kann auf eine aufwendige und/oder kostenintensive Schwingungsisolation verzichtet werden.

Bei der Erfindung wird mittels der Mikroskopiereinrichtung die Position und/oder eine Positionsveränderung von Granula der Immunzellen direkt optisch beobachtet. Insbesondere sind Granula mittels der Mikroskopiereinrichtung sichtbare, körnchenförmige Einlagerungen in Immunzellen. Die Freisetzung von Granula aus den Immunzellen, nennt man Degranulation. Es werden Granula in verschiedenen Ebenen oder Höhenebenen beobachtet. In dem mindestens einen Reaktionsraum können sich mehrere Immunzellen aufhalten. Die Immunzellen können in unterschiedlichen Höhen oder Höhenebenen angeordnet sein. Um eine allergische Reaktion einer Vielzahl von Granula, insbesondere unterschiedlicher Immunzellen, in demselben Reaktionsraum beobachten zu können, wird der Reaktionsraum in vorgegebenen, unterschiedlichen Höhenebenen mittels der Mikroskopiereinrichtung beobachtet. Hierbei kann die Beobachtung in den unterschiedlichen Höhenebenen in einer vorgegebenen zeitlichen Abfolge durchgeführt werden. Hierzu kann eine hoch auflösende Mikroskopie eingesetzt werden. Vorzugsweise wird mit der Mikroskopiereinrichtung eine, insbesondere digitale, holographische Mikroskopie oder eine Shearographie realisiert. Die Mikroskopiereinrichtung kann insbesondere zum Erzeugen eines holographischen Bildes ausgebildet sein. Hierbei kann Shearographie eine Kurzbezeichnung für eine Shearing Interferometrie und/oder eine Laser Speckle Shearing Interferometrie sein. Hierbei handelt es sich um ein an sich bekanntes kohärent optisches Messverfahren. Insbesondere ist anstelle eines Lasers eine LED (LED: Licht-Emitierende-Diode) einsetzbar. Die digitale holographische Mikroskopie nutzt das Prinzip der Holographie, um ein Bild zu erzeugen. Hierbei kann mittels einer Lichtquelle, insbesondere einer LED oder eines Lasers, die zu untersuchende Blutprobe bzw. die zu untersuchenden Immunzellen beleuchtet werden. Das hierbei gestreute Licht kann mit Licht einer Referenzquelle derselben Lichtquelle, insbesondere derselben LED oder desselben Lasers, interferieren. Hier kann die Referenzquelle mittels des Referenzraumes bereitgestellt sein. Das hierbei gebildete Interferenzmuster kann die direkte optische Beobachtung ermöglichen. Insbesondere kann das Interferenzmuster mittels eines, vorzugsweise digitalen, Sensors aufgenommen werden. Vorzugsweise ermöglicht die Mikroskopiereinrichtung die Ermittlung eines quantitativen Phasenkontrastes.

Nach einer weiteren, auch eigenständig und unabhängig von der vorliegenden Erfindung denkbaren, Ausführungsform werden mittels der Mikroskopiereinrichtung lebende Immunzellen der Blutprobe identifiziert. Somit kann zwischen lebenden und toten Zellen unterschieden werden. Hierbei kann die Identifizierung lebender Immunzellen automatisiert durchgeführt werden. Eine automatisierte Identifizierung lebender Immunzellen ermöglicht eine erhebliche Zeit- und/oder Kostenreduktion. Vorzugsweise werden ausschließlich als lebend identifizierte Immunzellen bei der Beobachtung und/oder der Auswertung eine Reaktion der Immunzellen bei einer Kontaktierung mit dem mindestens einen Allergen berücksichtigt. Hierdurch lassen sich Fehler bei der Auswertung der Beobachtung reduzieren. Insbesondere wird vermieden, dass eine nicht beobachtete allergische Reaktion bei einer toten Immunzelle als eine nicht vorhandene allergische Sensibilisierung bzw. Allergie interpretiert wird. Vorzugsweise wird ein Anregungszustand von Immunzellen, insbesondere Basophilzellen, festgestellt. Hierzu kann die Mikroskopiereinrichtung als ein quantitatives Phasenkontrastmikroskop ausgebildet sein. Ein optischer Weg des Lichts durch mindestens eine Immunzelle und/oder eine Lichtabsorption mindestens einer Immunzelle kann gemessen und/oder ausgewertet werden. Insbesondere sind tote Immunzellen von lebenden Immunzellen aufgrund einer Messung des optischen Weges bzw. des Lichtweges unterscheidbar, da tote Immunzellen beim Sterben platzen und sich hierdurch eine Veränderung des optischen Weges bzw. des Lichtweges ergibt.

Bei der Erfindung wird eine Position von Granula in Immunzellen, eine Bewegung der Granula und/oder eine Degranulation für eine Beobachtungszeit von bis zu 10 Minuten oder von 60 Sekunden bis 300 Sekunden beobachtet. Die Beobachtungszeit kann mit dem Kontaktieren der Blutprobe mit dem mindestens einen Allergen oder dem Zuführen des mindestens eines Allergens in den mikrofluidischen Chip in Gang gesetzt werden. Während der Beobachtungszeit können mehrere Reaktionsräume eines mikrofluidischen Chips in vorgegebenen Zeitabständen mehrfach aufeinanderfolgend beobachtet werden. Insbesondere können während der Beobachtungszeit mehrere Ebenen oder Höhenebenen in jeweils einem Reaktionsraum in vorgegebenen Zeitabständen mehrfach aufeinanderfolgend beobachtet werden. Somit ist es nicht notwendig, dass ein einzelner Reaktionsraum und/oder eine einzelne Ebene innerhalb eines einzelnen Reaktionsraumes ununterbrochen über die gesamte Beobachtungszeit beobachtet wird. Stattdessen kann es ausreichen, innerhalb der Beobachtungszeit mehrere Beobachtungen und/oder Aufnahmen zu machen, um auf der Basis einer Folge von Beobachtungen und/oder Aufnahmen eine Auswertung durchführen zu können.

Vorzugsweise wird die Beobachtung und/oder Auswertung automatisiert durchgeführt. Hierdurch lässt sich der Zeit- und/oder Kostenaufwand reduzieren. Insbesondere wird eine digitale Bildaufzeichnung und/oder Bildaufnahme zum Beobachten einer Reaktion der Blutprobe, insbesondere der Immunzellen, auf das Kontaktieren mit dem mindestens einen Allergen eingesetzt. Beispielsweise kann eine Bildverarbeitungssoftware zum Bereitstellen und/oder Auswerten von Bildaufnahmen verwendet werden. Hierdurch lässt sich eine möglicherweise vorhandene allergische Sensibilisierung bzw. Allergie in Bezug zu verschiedenen Allergenen innerhalb einer vergleichsweise kurzen Zeit testen. Ein entsprechender Allergietest kann nach der Erfindung in einer Zeit von weniger als 10 Minuten durchgeführt werden.

Es ergibt sich ein Verfahren zum Bestimmen einer Degranulation bei Immunzellen mit den Merkmalen des hier beschriebenen Verfahrens zum Durchführen eines Allergietests. Hierbei wird eine Degranulation oder ein Ausbleiben der Degranulation mittels einer Mikroskopiereinrichtung direkt optisch beobachtet. Mittels der Mikroskopiereinrichtung kann die Position und/oder eine Positionsveränderung von Granula der beobachteten Zellen direkt optisch bestimmt werden. Dabei erfolgt die Bestimmung einer Degranulation aufgrund einer Beobachtung und/oder Messung eines quantitativen Phasenkontrastes. Hierbei kann ein quantitativer Phasenkontrast von mindestens einer Zelle mittels einer interferometrischen Messung von sich überlagerndem Licht bestimmt oder gemessen werden. Ein erster Teil des sich überlagernden Licht kann durch einen Reaktionsraum mit der mindestens einen Zelle und ein weiterer Teil des sich überlagernden Lichts kann durch einen Referenzraum ohne Zellen hindurchlaufen. Die Mikroskopiereinrichtung kann zum Erzeugen eines holographischen Bildes ausgebildet sein.

Des Weiteren ist eine Vorrichtung zum Durchführen eines Allergietests mit einem erfindungsgemäßen Verfahren von Vorteil, wobei die Vorrichtung eine Mikroskopiereinrichtung und einen mindestens teilweise transparenten mikrofluidischen Chip zum direkten und/oder optischen Beobachten einer Degranulation aufweist und die Mikroskopiereinrichtung zum Erzeugen eines holographischen Bildes ausgebildet ist. Gegebenenfalls kann die Mikroskopiereinrichtung nur relevante Teile, insbesondere eine Objektiveinrichtung, eine Sensoreinrichtung und/oder eine Linseneinrichtung, einer üblichen Mikroskopiereinrichtung aufweisen. Hierdurch kann eine besonders kompakte Ausbildung oder Gestaltung der Vorrichtung realisierbar sein. Mittels der Vorrichtung ist eine allergische Reaktion oder ein Ausbleiben einer allergischen Reaktion beobachtbar.

Der mikrofluidische Chip hat mindestens einen Reaktionsraum, insbesondere mehrere Reaktionsräume. Mindestens ein, zwei oder mehrere Mikrokanäle können in den Reaktionsraum münden. Mehrere Reaktionsräume können mittels Trennelementen oder Wänden voneinander getrennt sein. Insbesondere weist der mikrofluidische Chip im Bereich des mindestens einen Reaktionsraumes transparente Fensterflächen auf. Die transparenten Fensterflächen können auf zwei voneinander abgewandten Seiten des mikrofluidischen Chips angeordnet sein. Die transparenten Fensterflächen können quer oder rechtwinklig zu einer optischen Achse, einem optischen Weg und/oder einem Lichtweg der Vorrichtung und/oder der Mikroskopiereinrichtung ausgerichtet sein. Insbesondere sind die transparenten Fensterflächen derart zueinander angeordnet, dass ein optischer Weg, ein Lichtweg, eine Lichtwelle und/oder eine elektromagnetische Welle durch eine erste Fensterfläche hindurch in den Reaktionsraum und durch eine zweite Fensterfläche hindurch aus dem Reaktionsraum heraustreten kann. Vorzugsweise ist jedem Reaktionsraum ein Referenzraum zugeordnet. Der Referenzraum kann transparente Fensterflächen aufweisen. Insbesondere sind transparente Fensterflächen des Referenzraumes auf zwei voneinander abgewandten Seiten des mikrofluidischen Chips angeordnet. Vorzugsweise sind die transparenten Fensterflächen des Reaktionsraumes zugleich auch die transparenten Fensterflächen des zugehörigen Referenzraumes. Die Fensterflächen können jeweils mittels einer Glasschicht gebildet sein. Hierbei kann eine einzige oder einzelne Glasschicht eine Seite des mikrofluidischen Chips abdecken.

Vorzugsweise hat der mindestens eine Reaktionsraum und/oder der Referenzraum eine Grundfläche oder auf zwei voneinander abgewandten Seiten jeweils eine transparente Fensterfläche im Bereich von etwa 100 µm x 100 µm. Insbesondere beträgt die Grundfläche des mindestens einen Reaktionsraumes und/oder des Referenzraumes oder die transparente Fensterfläche im Bereich des mindestens einen Reaktionsraumes und/oder im Bereich des Referenzraumes weniger als 50.000 µm², weniger als 20.000 µm² oder etwa 10.000 µm². Insbesondere hat der mindestens eine Reaktionsraum und/oder der Referenzraum eine Höhe von weniger als 1 mm und/oder weniger als 100 µm.

Nach einer weiteren Ausführungsform weist die Mikroskopiereinrichtung ein, insbesondere digitales, holographisches Mikroskop oder ein Shearographie-Mikroskop auf. Vorzugsweise hat die Mikroskopiereinrichtung ein quantitatives Phasenkontrastmikroskop. Insbesondere ist der mikrofluidische Chip zwischen einer Lichtquelle und einer Objektiveinrichtung angeordnet. Die Lichtquelle kann als eine LED oder ein Laser ausgebildet sein. Der mikrofluidische Chip kann an einer Trageinrichtung befestigt sein. Die Lichtquelle einerseits und die Objektiveinrichtung und/oder die Mikroskopiereinrichtung andererseits können, ausgehend von dem mikrofluidischen Chip, in zwei voneinander abgewandten Bereichen angeordnet sein.

Des Weiteren kann die Objektiveinrichtung ein Bestandteil der Mikroskopiereinrichtung und/oder zwischen der Mikroskopiereinrichtung und dem mikrofluidischen Chip angeordnet sein. Vorzugsweise ist die Mikroskopiereinrichtung mit einem Computer verbunden. Hierbei kann der Computer zum Darstellen, Aufzeichnen, Speichern und/oder Auswerten von Bildern und/oder Daten ausgebildet sein. Insbesondere lässt sich mittels eines Computers eine automatisierte Beobachtung und/oder Auswertung realisieren.

Der mikrofluidische Chip kann Elektroden zum elektrophoretischen und/oder dielektrophoretischen Positionieren von Zellen, insbesondere Immunzellen, aufweisen. Hierbei kann das Positionieren der Zellen bzw. Immunzellen in mindestens einem Reaktionsraum des mikrofluidischen Chips erfolgen. Der mikrofluidische Chip hat mindestens einen Referenzraum. Insbesondere ist jedem Reaktionsraum jeweils ein Referenzraum zugeordnet. Insbesondere ist aufgrund einer Gestalt und/oder Ausrichtung mindestens einer Elektrode benachbart zu dem Reaktionsraum der Referenzraum gebildet. Vorzugsweise ist die Elektrode strangförmig und weist zum Ausbilden des mindestens einen Referenzraumes eine Umlenkung und/oder einen Bogen auf. Somit ist mittels mindestens einer Elektrode eine Doppelfunktion realisierbar. Hierbei bewirkt die mindestens eine Elektrode zum einen ein zuverlässiges Positionieren und/oder Halten von Zellen bzw. Immunzellen in dem Reaktionsraum. Zugleich ist aufgrund der geeigneten Ausbildung oder Gestalt der mindestens einen Elektrode der mindestens eine Referenzraum gebildet. Hierbei gewährleistet die Elektrode, dass keine Zellen bzw. Immunzellen, insbesondere nach dem Positionieren der Zellen in dem Reaktionsraum, in den Referenzraum gelangen. Vorzugsweise ist der Referenzraum zwischen einer ersten Elektrode und mindestens einer weiteren Elektrode gebildet.

Von besonderem Vorteil ist eine Verwendung eines erfindungsgemäßen Verfahrens, einer erfindungsgemäßen Vorrichtung und/oder eines erfindungsgemäßen mikrofluidischen Chips zum Durchführen eines Allergietests. Insbesondere stellt die Visualisierung bzw. die quantitative Erfassung der Degranulation als einen allergieauslösenden Prozess im Gegensatz zu bekannten Allergietests eine direkte Methode dar. Hierdurch kann die Anzahl fehlerhafter Auswertungen erheblich reduziert werden. Des Weiteren ist der zeitliche Aufwand erheblich reduzierbar, da bei einem Vorliegen einer allergischen Sensibilität eine entsprechende allergische Reaktion in einem Zeitraum von wenigen Minuten, insbesondere innerhalb von 60 Sekunden bis 300 Sekunden, beobachtbar ist. Schließlich können mittels einer vergleichsweise geringen Blutprobenmenge umfangreiche Tests mit den bisher verwendeten, bekannten und üblichen Allergenen durchgeführt werden.

Nachfolgend wird die Erfindung anhand der Figuren näher erläutert. Es zeigen:
- Fig. 1: eine schematische Seitenansicht einer erfindungsgemäßen Vorrichtung,
- Fig. 2: einen schematischen Ausschnitt einer Draufsicht auf einen erfindungsgemäße mikrofluidischen Chip, und
- Fig. 3: ein schematisches Ablaufdiagramm für ein erfindungsgemäßes Verfahren.

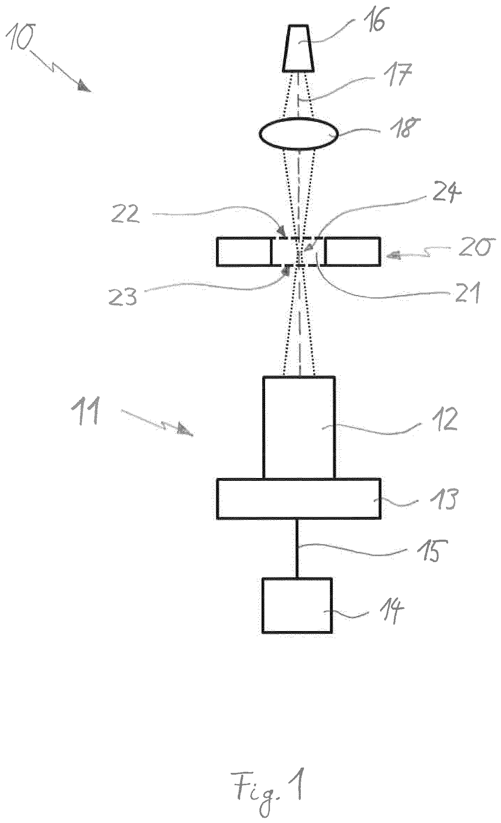

Fig. 1 zeigt eine schematische Seitenansicht einer erfindungsgemäßen Vorrichtung 10. Die Vorrichtung 10 weist eine Mikroskopiereinrichtung 11 auf. Bei diesem Ausführungsbeispiel ist die Mikroskopiereinrichtung 11 als ein digitales holographisches Mikroskop ausgebildet. Insbesondere kann die Mikroskopiereinrichtung 11 dabei zum Erzeugen eines holographischen Bildes ausgebildet sein. Des Weiteren weist die Mikroskopiereinrichtung 11 bei diesem Ausführungsbeispiel eine Objektiveinrichtung 12 und eine Sensoreinrichtung 13 auf. Die Sensoreinrichtung 13 kann als ein Detektor und/oder als ein Bildsensor, insbesondere ein CCD-Sensor, ausgebildet sein.

Die Mikroskopiereinrichtung 11 ist mit einem Computer 14 verbunden. Hierdurch sind Daten der Mikroskopiereinrichtung 11 bzw. der Sensoreinrichtung 13 mittels einer Datenleitung 15 an den Computer 14 übermittelbar. Des Weiteren kann mittels des Computers 14 die Mikroskopiereinrichtung 11 gesteuert werden.

Die Vorrichtung 10 weist eine Lichtquelle 16 auf. Die Lichtquelle 16 ist bei diesem Ausführungsbeispiel als eine LED ausgebildet. Alternativ kann die Lichtquelle 16 ein Laser sein. Bei dieser schematischen Darstellung ist die Lichtquelle 16 derart angeordnet, dass eine optische Achse 17 in Richtung der Mikroskopiereinrichtung 11 ausgerichtet ist. Hier ist die optische Achse 17 als eine gestrichelte Linie dargestellt. Des Weiteren kann ein hier nicht näher dargestellter räumlicher Modulator, insbesondere ein sogenannter SLM (Spatial Light Modulator), für das Licht der Lichtquelle 16 vorhanden sein.

Die Vorrichtung 10 hat eine Linseneinrichtung 18. Bei diesem Ausführungsbeispiel ist die Linseneinrichtung als eine Linsen-Filtereinrichtung ausgebildet. Die Linseneinrichtung 18 ist zum Fokussieren und/oder Filtern eines Lichtstrahls 19 ausgebildet. Hier ist der Lichtstrahl 19 mittels punktierter Linien schematisch dargestellt. Die Linseneinrichtung 18 kann eine oder mehrere Linsen aufweisen. Des Weiteren ist die Linseneinrichtung 18 zwischen der Lichtquelle 16 und der Mikroskopiereinrichtung 11 auf der optischen Achse 17 angeordnet. Bei diesem Ausführungsbeispiel ist die Linseneinrichtung 18 zum Einstellen bzw. Verändern des Fokus steuerbar. Beispielsweise kann die Linseneinstellung 18 mittels des Computers 14 gesteuert werden.

Schließlich weist die Vorrichtung 10 einen mikrofluidischen Chip 20 auf. Der mikrofluidische Chip 20 kann mittels einer hier nicht näher dargestellten Trageinrichtung positioniert und/oder gehalten sein. Der mikrofluidische Chip 20 ist zwischen der Lichtquelle 16 und der Mikroskopiereinrichtung 11 angeordnet. Hier ist der mikrofluidische Chip 20 auf der optischen Achse 17 zwischen der Linseneinrichtung 18 und der Objektiveinrichtung 12 positioniert. Der mikrofluidische Chip 20 ist mindestens teilweise transparent ausgebildet. Hierdurch kann der Lichtstrahl 19, ausgehend von der Lichtquelle 16, durch den mikrofluidischen Chip 20 zu der Mikroskopiereinrichtung 11 geführt werden. Alternativ kann der mirkofluidische Chip 20 lediglich einseitig transparent ausgebildet sein, wobei die dann die Bestrahlung und die Beobachtung bzw. Messung von derselben Seite aus erfolgt.

Der mikrofluidische Chip 20 weist mindestens einen Reaktionsraum 21 auf. Mindestens im Bereich des mindestens einen Reaktionsraumes 21 hat der mikrofluidische Chip 20 transparente Fensterflächen 22, 23. Die Fensterflächen 22, 23 sind an zwei voneinander abgewandten Seiten des mikrofluidischen Chips 20 angeordnet. Die Ebene des mikrofluidischen Chips 20 bzw. der Fensterflächen 22, 23 ist quer, bei diesem Ausführungsbeispiel im Wesentlichen rechtwinklig, zur optischen Achse 17 ausgerichtet.

Ein Fokus 24 des Lichtstrahls 19 ist innerhalb des Reaktionsraumes 21 positioniert. Mittels einer geeigneten Steuerung, insbesondere des Computers 14, kann die Lage des Fokus 24 innerhalb des mindestens einen Reaktionsraumes 21 verändert werden. Beispielsweise kann der Fokus 24 im Wesentlichen in Längsrichtung der optischen Achse 17 verschoben werden. Hierdurch können unterschiedliche Ebenen bzw. Höhenebenen innerhalb des mindestens einen Reaktionsraumes 21 beobachtet werden.

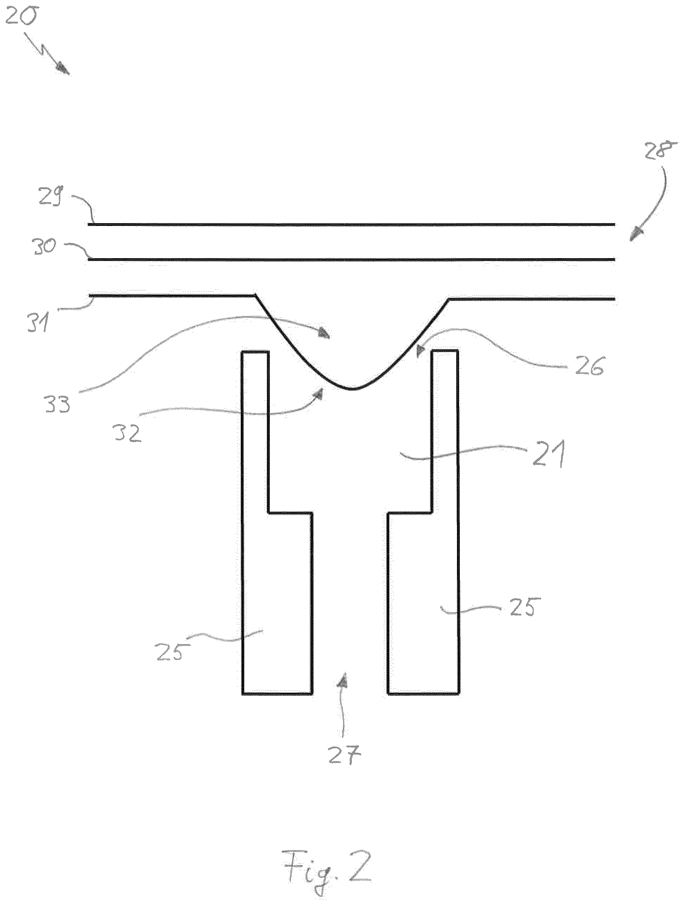

Fig. 2 zeigt einen schematischen Ausschnitt eines erfindungsgemäßen mikrofluidischen Chips 20. Hierbei ist der schematische Ausschnitt als eine Draufsicht dargestellt. Der mikrofluidische Chip 20 weist mehrere Reaktionsräume 21 auf, wobei hier jedoch nur ein einzelner Reaktionsraum 21 gezeigt ist. Der mikrofluidische Chip 20 hat mehrere Trennelemente 25. Mittels der Trennelemente 25 ist die Position und/oder Größe des mindestens einen Reaktionsraumes 21 definierbar. Insbesondere dienen die Trennelemente 25 zum Separieren von mehreren Reaktionsräumen 21. Bei diesem Ausführungsbeispiel sind die Trennelemente 25 als Trennwände ausgebildet.

Der Reaktionsraum 21 weist eine Zugangsöffnung 26 auf. Mittels der Zugangsöffnung 26 können hier nicht näher dargestellte Zellen oder Immunzellen einer Blutprobe in den Reaktionsraum 21 gelangen. Dabei kann der Begriff Blutprobe für eine direkte Blutprobe stehen oder für ein Serum aus einer direkten Blutprobe, das mit Zellen von speziellen Zelllinien versetzt oder in Kontakt gebracht wurde.

Des Weiteren weist der mikrofluidische Chip 20 mindestens einen Mikrokanal 27 auf. Insbesondere ist jeder Reaktionsraum 21 mit mindestens einem Mikrokanal 27 verbunden. Mittels des Mikrokanals 27 ist mindestens ein hier nicht näher dargestelltes Allergen in den Reaktionsraum 21 führbar. Bei diesem Ausführungsbeispiel sind die Zugangsöffnungen 26 und der Mikrokanal 27 an voneinander abgewandt liegenden Seiten des Reaktionsraumes 21 angeordnet. Des Weiteren sind bei diesem Ausführungsbeispiel sowohl die Zugangsöffnung 26 als auch der Mikrokanal 27 mittels zweier parallel zueinander angeordneter und spiegelsymmetrisch zueinander angeordneter Trennelemente 25 ausgebildet.

Der mikrofluidische Chip 20 weist eine dielektrophoretische Positioniereinrichtung 28 auf. Die dielektrophoretische Positioniereinrichtung 28 hat mehrere Elektroden 29, 30, 31. Die Elektroden 29, 30, 31 sind im Wesentlichen strangförmig ausgebildet. Des Weiteren sind die Elektroden 29, 30, 31 im Wesentlichen parallel zueinander ausgerichtet. Die dielektrophoretische Positioniereinrichtung 28 bzw. die Elektroden 29, 30, 31 sind derart angeordnet oder ausgebildet, dass Zellen bzw. Immunzellen dielektrophoretisch in dem mindestens einen Reaktionsraum 21 positionierbar sind. Hierbei weist die zu dem Reaktionsraum 21 nächstliegende Elektrode 31 im Bereich des Reaktionsraumes 21 bzw. der Zugangsöffnung 26 eine Umlenkung 32 auf. Die Umlenkung 32 ist in Richtung des Reaktionsraumes 21 bzw. der Zugangsöffnung 26 ausgebildet. Bei diesem Ausführungsbeispiel ist die Umlenkung 32 als eine Art Auswölbung der Elektrode 31 realisiert. Alternativ kann die Umlenkung 32 eine im Wesentlichen C-, U- oder V-förmige Ausbildung aufweisen. Bei diesem Ausführungsbeispiel ragt die Umlenkung 32 teilweise in den Bereich der Zugangsöffnung 26 hinein. Bei mehreren nebeneinander angeordneten Reaktionsräumen 21 kann die am nächsten zu den Reaktionsräumen 21 angeordnete Elektrode mäanderartig ausgebildet sein. Hierbei ergibt sich jeweils im Bereich der Reaktionsräume 21 eine Wölbung in Richtung des Reaktionsraumes 21 und in Bereichen der Trennelemente 25 jeweils eine Wölbung von den Trennelementen 25 weg.

Aufgrund der Umlenkung 32 ist zwischen der die Umlenkung 32 aufweisenden Elektrode 31 und der hierzu nächst benachbarten Elektrode 30 ein Referenzraum 33 gebildet. Aufgrund der dielektrophoretischen Wirkung der Positioniereinrichtung 28 ist realisierbar, dass keine Zellen bzw. Immunzellen innerhalb des Referenzraumes 33 und nur in dem Reaktionsraum 21 positionierbar sind. Zugleich kann mindestens ein Allergen mittels des Mikrokanals 27 sowohl in den Reaktionsraum 21 als auch in den Referenzraum 33 geführt werden. Alternativ kann das mindestens eine Allergen nur in den Reaktionsraum 21 und nicht in den Referenzraum 33 geleitet werden.

Der Reaktionsraum 21 und der Referenzraum 33 sind mittels Fensterflächen 22, 23, wie in Figur 1 gezeigt, direkt und/oder optisch beobachtbar. Der Referenzraum 33 ermöglicht eine Beobachtung von sich überlagerndem Licht, das durch den Reaktionsraum 21 bzw. durch den Referenzraum 33 hindurch läuft. Ein erster Teil des sich überlagernden Licht kann durch den Reaktionsraum 21 und ein weiterer Teil des sich überlagernden Lichts kann durch den Referenzraum 33 hindurchlaufen. Hierdurch ist eine quantitative Phasenkontrastmikroskopie ermöglicht.

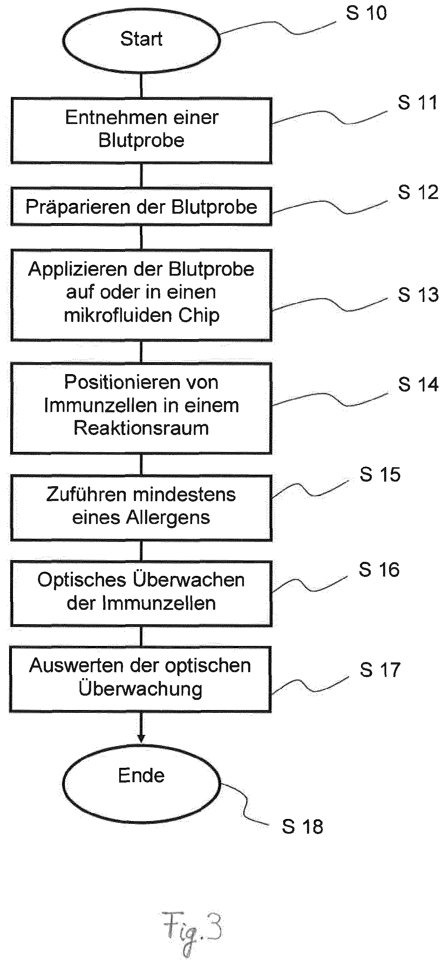

Fig. 3 zeigt ein schematisches Ablaufdiagramm für ein erfindungsgemäßes Verfahren. Nachfolgend wird das Verfahren unter Berücksichtigung der Vorrichtung 10 und des mikrofluidischen Chips 20 gemäß Figuren 1 und 2 näher erläutert.

Nach einem Start des Verfahrens gemäß Schritt S 10 wird mit Schritt S 11 eine Blutprobe entnommen. Beispielsweise kann eine Blutprobe von einer Person bzw. einem Patienten in üblicher Weise mittels einer Nadel bzw. Spritze entnommen werden. Hierbei reichen jedoch für das erfindungsgemäße Verfahren vergleichsweise geringe Blutmengen aus. Insbesondere ist eine Blutprobenmenge von weniger als 50 ml, weniger als 20 ml oder weniger als 1 ml ausreichend.

Anschließend wird die Blutprobe gemäß Schritt S 12 präpariert. Bei diesem Ausführungsbeispiel wird im Rahmen der Präparation der Blutprobe ein Zusatzmittel der Blutprobe zugeführt, um eine Blutgerinnung zu vermeiden. Des Weiteren wird im Rahmen der Präparation der Blutprobe der prozentuale Anteil der zu untersuchenden Immunzellen in der Blutprobe erhöht. Bei diesem Ausführungsbeispiel werden hierzu rote Blutkörperzellen mittels an sich bekannter Verfahren aus der Blutprobe entfernt. Des Weiteren können nicht relevante weiße Blutkörperzellen ebenfalls mit an sich bekannten Verfahren entfernt werden. Bei diesem Ausführungsbeispiel erfolgt eine Isolierung oder Erhöhung des Anteils von basophilen Zellen in der Blutprobe. Vorliegend ist eine Erhöhung des Anteils von basophilen Zellen in der Blutprobe in einem Bereich von 10 % bis 20 % ausreichend. Anschließend wird die Blutprobe bzw. werden die Zellen in einem geeigneten Medium suspendiert, um die Zellen für eine vorgegebene Zeit, insbesondere bis zu 24 Stunden, am Leben zu erhalten.

Anschließend wird die Blutprobe gemäß Schritt S 13 auf oder in einen mikrofluiden Chip 20 appliziert. Hierbei kann der mikrofluidische Chip 20 derart ausgebildet sein, dass die Blutprobe bzw. die zu untersuchenden Immunzellen mittels Kapilarkräften in den mikrofluidischen Chip 20 geführt werden. Alternativ kann die Blutprobe bzw. können die Immunzellen mittels einer geeigneten Einrichtung in den mikrofluidischen Chip 20 gepumpt werden.

Sodann erfolgt ein Positionieren von Immunzellen in mindestens einem Reaktionsraum 21 gemäß Schritt S 14. Hierbei kann das Positionieren mittels geeignet ausgebildeter Zellenfallen, Mikrokanäle, einer elektrophoretischen oder dielektrophoretischen Positioniereinrichtung 28 erfolgen. Bei diesem Ausführungsbeispiel werden jeweils mehrere Immunzellen in einem Reaktionsraum 21 mittels der dielektrophoretischen Positioniereinrichtung 28 angeordnet. Des Weiteren werden Immunzellen in mehrere Reaktionsräume 21 geführt und dort mittels der dielektrophoretischen Positioniereinrichtung 28 gehalten.

Anschließend wird gemäß Schritt S 15 mindestens ein Allergen zugeführt. Bei diesem Ausführungsbeispiel wird mindestens ein Allergen, insbesondere in flüssiger Form, mittels des Mikrokanals 27 in den Reaktionsraum 21 und den zugehörigen Referenzraum 33 geführt.

Gemäß Schritt S 16 wird ein optisches Überwachen der Immunzellen durchgeführt. Hierbei kann das optische Überwachen bereits vor dem Zuführen des mindestens einen Allergens, zusammen mit dem Zuführen des mindestens einen Allergens oder unmittelbar nach dem Zuführen des mindestens einen Allergens, gestartet werden. Bei diesem Ausführungsbeispiel erfolgt ein optisches Überwachen mittels der Mikroskopiereinrichtung 11. Bei diesem Ausführungsbeispiel ist die optische Überwachung als eine quantitative Phasenkontrastmikroskopie realisiert. Hierbei werden mittels der Mikroskopiereinrichtung 11 Granula der Immunzellen direkt und/oder optisch beobachtet. Insbesondere wird für eine vorgegebene Zeit von 60 Sekunden bis 300 Sekunden oder länger beobachtet, ob nach einer Kontaktierung der Immunzellen mit dem mindestens einen Allergen eine Degranulation erfolgt. Hierzu kann die Mikroskopiereinrichtung 11 eine digitale Bildaufzeichnung bzw. eine digitale Bildaufnahme ermöglichen. Im Rahmen der durchgeführten Beobachtung bzw. Überwachung werden mehrere Immunzellen in unterschiedlichen Ebenen bzw. Höhenebenen des mindestens einen Reaktionsraumes 21 beobachtet. Dazu kann die Mikroskopiereinrichtung 11 insbesondere zum Erzeugen eines holographischen Bildes ausgebildet sein.

Anschließend erfolgt gemäß Schritt S 17 eine Auswertung der optischen Beobachtung bzw. Überwachung. Sofern nach dem Kontaktieren der Immunzellen mit dem mindestens einen Allergen eine Degranulation beobachtet wird, wird dies als eine allergische Reaktion klassifiziert. Findet dagegen keine beobachtbare Degranulation statt, wird dies als eine nicht-allergische Reaktion aufgefasst.

Sodann endet das Verfahren gemäß Schritt S 18.

### Bezugszeichenliste:

- 10: Vorrichtung
- 11: Mikroskopiereinrichtung
- 12: Objektiveinrichtung
- 13: Sensoreinrichtung
- 14: Computer
- 15: Datenleitung
- 16: Lichtquelle
- 17: optische Achse
- 18: Linseneinrichtung
- 19: Lichtstrahl
- 20: mikrofluidischer Chip
- 21: Reaktionsraum
- 22: Fensterfläche
- 23: Fensterfläche
- 24: Fokus
- 25: Trennelement
- 26: Zugangsöffnung
- 27: Mikrokanal
- 28: dielektrophoretische Positioniereinrichtung
- 29: Elektrode
- 30: Elektrode
- 31: Elektrode
- 32: Umlenkung
- 33: Referenzraum

## Patentansprüche

1. Verfahren zum Durchführen eines Allergietests, bei dem eine Blutprobe mit mindestens einem Allergen in vitro kontaktiert wird, und bei dem mindestens eine allergische Reaktion oder ein Ausbleiben der mindestens einen allergischen Reaktion von Immunzellen mittels einer Mikroskopiereinrichtung (11) direkt optisch beobachtet wird, **dadurch gekennzeichnet, dass** bei der mindestens einen allergischen Reaktion eine Degranulation der Immunzellen beobachtet wird, dass die Immunzellen in unterschiedlichen Höhen oder Höhenebenen angeordnet sein können, dass mittels der Mikroskopiereinrichtung (11) die Position von Granula beobachtet wird, wobei die Granula in verschiedenen Ebenen oder Höhenebenen beobachtet werden, und dass eine Position von Granula in Immunzellen, eine Bewegung der Granula und/oder eine Degranulation für eine Beobachtungszeit von bis zu 10 Minuten oder 60 Sekunden bis 300 Sekunden beobachtet wird.

2. Verfahren nach Anspruch 1, **dadurch gekennzeichnet, dass** bei der einen allergischen Reaktion eine Degranulation der Granulozyten, basophilen Zellen, basophilen Granulozyten und/oder Mastzellen, der Blutprobe mit der Mikroskopiereinrichtung (11) beobachtet wird, vorzugsweise wird aufgrund der direkten optischen Beobachtung der Immunzellen bei Kontaktierung mit dem mindestens einen Allergen ein Ausbleiben der Degranulation als nicht-allergische Reaktion und ein Stattfinden der Degranulation als eine allergische Reaktion klassifiziert.

3. Verfahren nach Anspruch 1 oder 2, **dadurch gekennzeichnet, dass** nach der Entnahme der Blutprobe und vor dem Kontaktieren mit dem mindestens einen Allergen die Blutprobe zum Erhöhen des Anteils an Immunzellen präpariert wird, insbesondere wird der prozentuale Anteil von Immunzellen in der Blutprobe auf mehr als 5%, mehr als 20% oder höher erhöht, vorzugsweise wird der Anteil an roten Blutzellen und/oder an Immunzellen, insbesondere Granulozyten, basophilen Zellen, basophilen Granulozyten und/oder Mastzellen, verschiedenen weißen Blutzellen reduziert.

4. Verfahren nach einem der vorhergehenden Ansprüche, **dadurch gekennzeichnet, dass** ein mikrofluidischer Chip (20) zum Kontaktieren der Blutprobe mit dem mindestens einen Allergen verwendet wird, insbesondere werden Immunzellen der Blutprobe in mindestens einem Reaktionsraum (21) des mikrofluidischen Chips (20) angeordnet, vorzugsweise ist der mindestens eine Reaktionsraum (21) und/oder mindestens ein Referenzraum (33) des mikrofluidischen Chips (20) mindestens teilweise transparent ausgebildet.

5. Verfahren nach Anspruch 4, **dadurch gekennzeichnet, dass** mindestens ein Allergen in den mindestens einen Reaktionsraum (21) geführt wird, insbesondere wird das mindestens eine Allergen sowohl in den mindestens einen Reaktionsraum (21) mit den Immunzellen als auch in einen Referenzraum (33) ohne Immunzellen geführt, vorzugsweise ist jedem Reaktionsraum (21) jeweils ein Referenzraum (33) zugeordnet.

6. Verfahren nach einem der Ansprüche 4 oder 5, **dadurch gekennzeichnet, dass** Immunzellen der Blutprobe mittels einer mikrofluidischen Zellenfalle, einer Elektrophorese und/oder einer Dielektrophorese auf oder in dem mikrofluidischen Chip (20) angeordnet werden, insbesondere werden Immunzellen mittels der Elektrophorese und/oder Dielektrophorese in mindestens einem Reaktionsraum (21) positioniert und/oder gehalten, vorzugsweise wird mindestens ein Referenzraum (33) mittels der Elektrophorese und/oder Dielektrophorese frei von Immunzellen bereit gestellt.

7. Verfahren nach einem der vorhergehenden Ansprüche, **dadurch gekennzeichnet, dass** mittels der Mikroskopiereinrichtung (11) die Position und/oder eine Positionsveränderung von Granula von Immunzellen direkt optisch beobachtet wird, vorzugsweise wird mit der Mikroskopiereinrichtung (11) eine, insbesondere digitale, holografische Mikroskopie, eine Shearografie und/oder eine quantitative Phasenkontrastmikroskopie realisiert.

8. Verfahren nach einem der vorhergehenden Ansprüche, **dadurch gekennzeichnet, dass** mittels der Mikroskopiereinichtung (11) lebende Immunzellen der Blutprobe identifiziert werden, insbesondere wird die Identifizierung lebender Immunzellen automatisiert durchgeführt, vorzugsweise werden ausschließlich als lebend identifizierte Immunzellen bei der Beobachtung und/oder der Auswertung einer Reaktion der Immunzellen bei einer Kontaktierung mit dem mindestens einen Allergen berücksichtigt.

9. Verfahren nach einem der vorhergehenden Ansprüche, **dadurch gekennzeichnet, dass** die Beobachtungszeit beim Beobachten der Position von Granula in Immunzellen, der Bewegung der Granula und/oder der Degranulation mit dem Kontaktieren der Blutprobe mit dem mindestens einen Allergen in Gang gesetzt wird, vorzugsweise werden während der Beobachtungszeit mehrere Reaktionsräume (21) eines mikrofluidischen Chips (20) in vorgegeben Zeitabständen mehrfach aufeinander folgend beobachtet.

10. Verfahren nach einem der vorhergehenden Ansprüche, **dadurch gekennzeichnet, dass** die Beobachtung und/oder Auswertung automatisiert durchgeführt wird, insbesondere wird eine digitale Bildaufzeichnung zum Beobachten einer Reaktion der Blutprobe auf das Kontaktieren mit dem mindestens einen Allergen und/oder eine Bildverarbeitungssoftware zum Bereitstellen und/oder Auswerten von Bildaufnahmen eingesetzt.

11. Verfahren zum Bestimmen einer Degranulation bei Immunzellen, bei dem eine Blutprobe mit mindestens einem Reaktionspartner in vitro kontaktiert wird, und bei dem eine Degranulation oder ein Ausbleiben der Degranulation mittels einer Mikroskopiereinrichtung (11) direkt optisch beobachtet wird, **dadurch gekennzeichnet, dass** die Immunzellen in unterschiedlichen Höhen oder Höhenenbenen angeordnet sein können, dass mittels der Mikroskopiereinrichtung (11) die Position von Granula beobachtet wird, wobei die Granula in verschiedenen Ebenen oder Höhenebenen beobachtet werden, und dass eine Position von Granula in Immunzellen, eine Bewegung der Granula und/oder eine Degranulation für eine Beobachtungszeit von bis zu 10 Minuten oder 60 Sekunden bis 300 Sekunden beobachtet wird, insbesondere erfolgt die Bestimmung der Degranulation aufgrund einer Beobachtung und/oder Messung eines quantitativen Phasenkontrastes.

12. Vorrichtung (10) zum Durchführen eines Allergietests mit einem Verfahren nach einem der vorhergehenden Ansprüche, mit einer Mikroskopiereinrichtung (11) und mit einem mindestens teilweise transparenten mikrofluidischen Chip (20) zum direkten optischen Beobachten einer allergischen Reaktion oder zum Beobachten des Ausbleibens einer allergischen Reaktion, **dadurch gekennzeichnet, dass** die Mikroskopiereinrichtung (11) zum Erzeugen eines holographischen Bildes ausgebildet ist und dass der mikrofluidische Chip (20) mindestens einen Reaktionsraum (21) und einen diesem zugeordneten Referenzraum (33) und eine mikrofluidische Zellenfalle oder Elektroden (29, 30, 31) zum elektrophoretischen und/oder dielektrophoretischen Positionieren von Immunzellen in dem mindestens einen Reaktionsraum (21) hat, und dass entweder keine Immunzellen innerhalb des Referenzraumes (33) positionierbar sind oder ein Allergen nicht in den Referenzraum (33) geleitet werden kann.

13. Vorrichtung (10) nach Anspruch 12, **dadurch gekennzeichnet, dass** der mikrofluidische Chip (20) mehrere Reaktionsräume (21), hat, und/oder der mikrofluidische Chip (20) im Bereich des mindestens einen Reaktionsraumes (21) transparente Fensterflächen (22, 23) auf zwei voneinander abgewandten Seiten aufweist, vorzugsweise ist jedem Reaktionsraum (21) ein Referenzraum (33), insbesondere mit transparenten Fensterflächen (22, 23) auf zwei voneinander abgewandten Seiten, zugeordnet.

14. Vorrichtung (10) nach Anspruch 13, **dadurch gekennzeichnet, dass** der mindestens eine Reaktionsraum (21) und/oder ein Referenzraum (33) eine Grundfläche oder auf zwei voneinander abgewandten Seiten jeweils eine transparente Fensterfläche (22, 23) von 100 µm mal 100 µm hat, insbesondere hat der mindestens eine Reaktionsraum (21) und/oder der Referenzraum (33) eine Höhe von weniger als 1 mm und/oder weniger als 100 µm.

15. Vorrichtung (10) nach einem der Ansprüche 12 bis 14, **dadurch gekennzeichnet, dass** die Mikroskopiereinrichtung (11) ein, insbesondere digitales, holografisches Mikroskop, ein Shearografie-Mikroskop und/oder ein Phasenkontrastmikroskop aufweist, insbesondere ist der mikrofluidische Chip (20) zwischen einer Lichtquelle (16) und einer Objektiveinrichtung (12) angeordnet, vorzugsweise ist die Mikroskopiereinrichtung (11) mit einem Computer (14) zum Darstellen, Aufzeichnen und/oder Auswerten von Bildern und/oder Daten verbunden.

16. Vorrichtung (10) nach einem der Ansprüche 12 bis 15, bei der aufgrund einer Gestalt und/oder Ausrichtung mindestens einer Elektrode (31) benachbart zu dem Reaktionsraum (21) der Referenzraum (33) gebildet wird, vorzugsweise ist die Elektrode (31) strangförmig und weist zum Ausbilden des Referenzraumes (33) eine Umlenkung (32) und/oder einen Bogen auf.

17. Verwendung einer Vorrichtung (10) nach einem der Ansprüche 12 bis 16 zum Durchführen eines Allergietests.

## Claims

1. Process for carrying out an allergy test, in which a blood sample with at least one allergen is contacted *in vitro*; and in which one allergic reaction or an absence of the at least one allergic reaction of immune cells is directly optically observed by means of a microscope (11), **characterised in that** in the at least one allergic reaction a degranulation of the immune cells is observed, the immune cells may be arranged in different heights or planes, the position of granules being observed by means of the microscope (11), wherein the granules are observed in various heights or height planes, and that a position of the granules in the immune cells, a movement and/or a degranulation of the granules is observed in an observation time of up to 10 minutes or 60 seconds to 300 seconds.

2. Process according to claim 1, **characterised in that** degranulation of granulocytes, basophilic cells, basophilic granulocytes and/or mast cells, of the blood sample is observed with the microscope (11) in case of an allergic reaction, preferably an absence of degranulation is classified as a non-allergic reaction and a presence of degranulation is classified as an allergic reaction on the basis of the directly optical observation of the immune cells on contacting with the at least one allergen.

3. Process according to claim 1 or 2, **characterised in that** after taking the blood sample and before contacting the blood sample with the at least one allergen, the blood sample is processed to increase the percentage of immune cells, preferably the percentage of immune cells in the blood sample is increased to more than 5%, more than 20% or higher, preferably the percentage of red blood cells and/or of immune cells, in particular reduces various white blood cells granulocytes, basophilic cells, basophilic granulocytes and/or mast cells.

4. Process according to one of the preceding claims, **characterised in that** a microfluidic chip (20) is used for contacting the blood sample with the at least one allergen, in particular immune cells of the blood sample are arranged in at least one reaction chamber (21) of the microfluidic chip (20), in particular the at least one reaction chamber (21) and/or at least one reference chamber (33) of the microfluidic chip (20) is configured to be at least partially transparent.

5. Process according to claim 4, **characterised in that** at least one allergen is fed into the at least one reaction chamber (21), in particular the at least one allergen is fed both into the at least one reaction chamber (21) with the immune cells and into a reference chamber (33) without immune cells, preferably a reference chamber (33) is associated with each reaction chamber (21).

6. Process according to one of claims 4 or 5, **characterised in that** immune cells of the blood sample are arranged on or in the microfluidic chip (20) by means of a microfluidic cell trap, electrophoresis and/or dielectrophoresis, in particular immune cells are positioned and/or held by means of electrophoresis and/or dielectrophoresis in at least one reaction chamber (21), preferably at least one reference chamber (33) is provided free from immune cells by means of electrophoresis and/or dielectrophoresis.

7. Process according to one of the preceding claims, **characterised in that** the position and/or a change in the position of granules of immune cells are observed directly optically by means of the microscope (11), preferably to realise an especially digital, holographic microscopy, a shearography and/or a quantitative phase contrast microscopy.

8. Process according to one of the preceding claims, **characterised in that** the live immune cells of the blood sample are identified by means of the microscope (11), the identification of live immune cells being carried out in an automated manner, and only immune cells identified as live immune cells are taken into account during the observation and/or the analysis of a reaction of the immune cells on contacting the immune cells with the at least one allergen.

9. Process according to one of the preceding claims, **characterised in that** the position of granules in immune cells, a motion of the granules and/or degranulation are observed, the observation time being started with the contacting of the blood sample with the at least one allergen, preferably a plurality of reaction chambers (21) of a microfluidic chip (20) are observed several times consecutively at predefined time intervals.

10. Process according to one of the preceding claims, **characterised in that** the observation and/or analysis are carried out in an automated manner, preferably using a digital image recording to observe a reaction of the blood sample upon contacting the blood sample with the at least one allergen and/or using an image processing software to provide and/or analyse recorded images.

11. Process for determining degranulation in immune cells, in which a blood sample is taken, and in which a blood sample is contacted *in vitro* with at least one reaction partner, and in which degranulation or the absence of degranulation is observed directly optically by means of a microscope (11), **characterised in that** the immune cells may be arranged in different heights or height planes, the position of the granules being observed by means of the microscope (11), wherein the granules are observed in different planes or height planes, and that a position of the granules in immune cells, a movement of the granules and/or a degranulation is observed in an observation period of up to 10 minutes or 60 seconds to 300 seconds, especially on the basis of an observation and/or measurement of a quantitative phase contrast.

12. Device (10) for carrying out an allergy test with a process according to one of the preceding claims, with a microscope (11) and with an at least partially transparent microfluidic chip (20) in order to directly optically observe an allergic reaction or to observe the absence of an allergic reaction, **characterised in that** the microscope (11) is configured to generate a holographic image, and that the microfluidic chip (20) has a reaction chamber (21) and an associated reference chamber (33) and a microfluidic cell trap or electrodes (29, 30, 31) for the electrophoretic and/or dielectrophoretic positioning of immune cells in the at least one reaction chamber (21), and that either no immune cells are placeable inside the reference chamber (33) or an allergen cannot be fed into the reference chamber (33).

13. Device (10) according to claim 12, **characterised in that** the microfluidic chip (20) has a plurality of reaction chambers (21), and/or the microfluidic chip (20) possesses transparent window surfaces (22, 23) in an area of the at least one reaction chamber (21) on two sides facing away from one another, preferably a reference chamber (33) is associated with each reaction chamber (21) in particular with transparent window surfaces (22, 23) on two sides facing away from one another.

14. Device (10) according to claim 13, **characterised in that** the at least one reaction chamber (21) and/or a reference chamber (33) has a base or a respective transparent window surface (22,23) of 100 µm x 100 µm on the two sides facing away from one another, in particular the at least one reaction chamber (21) and/or the reference chamber (33) has a height of less than 1 mm and/or less than 100 µm.

15. Device (10) according to one of claims 12 to 14, **characterised in that** the microscope (11) possesses a digital, holographic microscope, a shearography microscope and/or a phase contrast microscope, in particular the microfluidic chip (20) is arranged between a light source (16) and an objective lens device (12), preferably the microscope (11) is connected to a computer (14) for viewing, recording and/or analysing images and/or data.

16. Device (10) according to one of claims 12 to 15, in which based on a shape and/or orientation of at least one of the electrodes (31) is formed neighbouring the reaction chamber (21) of the reference chamber (33), preferably the electrode (31) is in the shape of a strand and has a deflection (32) and/or an arch to form the reference chamber (33).

17. Use of a device (10) according to one of claims 12 to 16 to carry out an allergy test.

## Revendications

1. Procédé de réalisation d'un test d'allergie, dans lequel un échantillon de sang est mis en contact avec au moins un allergène in vitro, et dans lequel au moins une réaction allergique ou une absence de ladite au moins une réaction allergique est observée directement de manière optique par des cellules immunitaires au moyen d'un dispositif de microscopie (11), **caractérisé en ce que**, lors de ladite au moins une réaction allergique, une dégranulation des cellules immunitaires est observée, **en ce que** les cellules immunitaires peuvent être disposées en différents plans ou à différents niveaux de hauteur, **en ce que** la position de granules est observée au moyen du dispositif de microscopie (11), les granules étant observés en différents plans ou à différents niveaux de hauteur, et **en ce qu'**une position de granules dans des cellules immunitaires, un mouvement des granules et/ou une dégranulation sont observés pour une durée d'observation allant jusqu'à 10 minutes ou de 60 à 300 secondes.

2. Procédé selon la revendication 1, **caractérisé en ce que**, lors d'une réaction allergique, on observe une dégranulation des granulocytes, des cellules basophiles, des granulocytes basophiles et/ou des mastocytes, de l'échantillon de sang avec le dispositif de microscopie (11), de préférence, sur la base de l'observation optique directe des cellules immunitaires lors de la mise en contact avec ledit au moins un allergène, une absence de dégranulation est classée comme une réaction non allergique et une survenue de la dégranulation est classée comme une réaction allergique.

3. Procédé selon la revendication 1 ou 2, **caractérisé en ce que**, après le prélèvement de l'échantillon de sang et avant la mise en contact avec ledit au moins un allergène, l'échantillon de sang est préparé pour augmenter la proportion de cellules immunitaires, plus précisément le pourcentage de cellules immunitaires dans l'échantillon de sang est augmenté à plus de 5 %, plus de 20 % ou plus, la proportion de globules rouges et/ou de cellules immunitaires, notamment de granulocytes, de cellules basophiles, de granulocytes basophiles et/ou de mastocytes, de différentes cellules sanguines blanches étant de préférence réduite.

4. Procédé selon l'une des revendications précédentes, **caractérisé en ce qu'**une puce microfluidique (20) est utilisée pour mettre en contact l'échantillon de sang avec ledit au moins un allergène, à savoir notamment que des cellules immunitaires de l'échantillon de sang sont disposées dans au moins un espace de réaction (21) de la puce microfluidique (20), de préférence ledit au moins un espace de réaction (21) et/ou au moins un espace de référence (33) de la puce microfluidique (20) est réalisé au moins partiellement transparent.

5. Procédé selon la revendication 4, **caractérisé en ce qu'**au moins un allergène est amené dans ledit au moins un espace de réaction (21), en particulier le dit au moins un allergène est amené aussi bien dans ledit au moins un espace de réaction (21) avec les cellules immunitaires que dans un espace de référence (33) sans cellules immunitaires, de préférence un espace de référence (33) est associé à chaque espace de réaction (21).

6. Procédé selon l'une des revendications 4 ou 5, **caractérisé en ce que** des cellules immunitaires de l'échantillon de sang sont disposées sur ou dans la puce microfluidique (20) au moyen d'un piège à cellules microfluidique, d'une électrophorèse et/ou d'une diélectrophorèse, en particulier des cellules immunitaires sont positionnées et/ou maintenues dans au moins un espace de réaction (21) au moyen de l'électrophorèse et/ou de la diélectrophorèse, de préférence au moins un espace de référence (33) est mis à disposition libre de cellules immunitaires au moyen de l'électrophorèse et/ou de la diélectrophorèse.

7. Procédé selon l'une des revendications précédentes, **caractérisé en ce qu'**au moyen du dispositif de microscopie (11), on observe directement de manière optique la position et/ou un changement de position de granules de cellules immunitaires, de préférence on réalise avec le dispositif de microscopie (11) une microscopie holographique, notamment numérique, une shearographie et/ou une microscopie quantitative à contraste de phase.

8. Procédé selon l'une des revendications précédentes, **caractérisé en ce que** des cellules immunitaires vivantes de l'échantillon de sang sont identifiées au moyen du dispositif de microscopie (11), en particulier l'identification de cellules immunitaires vivantes est effectuée de manière automatisée, de préférence seules les cellules immunitaires identifiées comme vivantes sont prises en compte lors de l'observation et/ou de l'évaluation d'une réaction des cellules immunitaires lors d'une mise en contact avec ledit au moins un allergène.

9. Procédé selon l'une des revendications précédentes, **caractérisé en ce que** le temps d'observation est déclenché lors de l'observation de la position de granules dans des cellules immunitaires, du mouvement des granules et/ou de la dégranulation avec la mise en contact de l'échantillon de sang avec ledit au moins un allergène, de préférence plusieurs espaces de réaction (21) d'une puce microfluidique (20) sont observés plusieurs fois de suite à des intervalles de temps prédéterminés pendant le temps d'observation.

10. Procédé selon l'une des revendications précédentes, **caractérisé en ce que** l'observation et/ou l'évaluation sont réalisées de manière automatisée, à savoir notamment qu'un enregistreur d'image numérique est utilisé pour observer une réaction de l'échantillon de sang à la mise en contact avec ledit au moins un allergène et/ou un logiciel de traitement d'image pour mettre à disposition et/ou évaluer des enregistrements d'image.

11. Procédé pour déterminer une dégranulation dans des cellules immunitaires, dans lequel un échantillon de sang est mis en contact in vitro avec au moins un partenaire de réaction, et dans lequel une dégranulation ou une absence de dégranulation est observée directement de manière optique au moyen d'un dispositif de microscopie (11), **caractérisé en ce que** les cellules immunitaires peuvent être disposées en différents plans ou à différents niveaux de hauteur, et **en ce qu'**une position de granules dans des cellules immunitaires, un mouvement des granules et/ou une dégranulation sont observés pendant une durée d'observation allant jusqu'à 10 minutes ou de 60 à 300 secondes au moyen d'un dispositif de microscopie (11),, à savoir notamment que la détermination de la dégranulation a lieu sur la base d'une observation et/ou d'une mesure d'un contraste de phase quantitatif.

12. Dispositif (10) pour la réalisation d'un test d'allergie avec un procédé selon l'une des revendications précédentes, avec un dispositif de microscopie (11) et une puce microfluidique (20) au moins partiellement transparente pour l'observation optique directe d'une réaction allergique ou pour l'observation de l'absence d'une réaction allergique, **caractérisé, en ce que** le dispositif de microscopie (11) est conçu pour produire une image holographique et **en ce que** la puce microfluidique (20) comprend au moins un espace de réaction (21) et un espace de référence (33) associé à celui-ci et un piège à cellules microfluidique ou des électrodes (29, 30, 31) pour le positionnement électrophorétique et/ou diélectrophorétique de cellules immunitaires dans ledit au moins un espace de réaction (21), et **en ce que** soit aucune cellule immunitaire ne peut être positionnée à l'intérieur de l'espace de référence (33), soit un allergène ne peut pas être dirigé vers l'espace de référence (33).

13. Dispositif (10) selon la revendication 12, **caractérisé en ce que** la puce microfluidique (20) a plusieurs espaces de réaction (21), et/ou la puce microfluidique (20) présente dans la zone de l'au moins un espace de réaction (21) des surfaces de fenêtre transparentes (22, 23) sur deux côtés opposés l'un à l'autre, de préférence à chaque espace de réaction (21) est associé un espace de référence (33), notamment avec des surfaces de fenêtre transparentes (22, 23) sur deux côtés opposés l'un à l'autre.

14. Dispositif (10) selon la revendication 13, **caractérisé en ce que** ledit au moins un espace de réaction (21) et/ou un espace de référence (33) a une surface de base ou sur deux côtés opposés l'un à l'autre respectivement une surface de fenêtre transparente (22, 23) de 100 µm par 100 µm, en particulier ledit au moins un espace de réaction (21) et/ou l'espace de référence (33) a une hauteur de moins de 1 mm et/ou de moins de 100 µm.

15. Dispositif (10) selon l'une des revendications 12 à 14, **caractérisé en ce que** le dispositif de microscopie (11) comporte un microscope holographique, notamment numérique, un microscope à shearographie et/ou un microscope à contraste de phase, notamment la puce microfluidique (20) est disposée entre une source lumineuse (16) et un dispositif d'objectif (12), de préférence le dispositif de microscopie (11) est relié à un ordinateur (14) pour représenter, enregistrer et/ou évaluer des images et/ou des données.

16. Dispositif (10) selon l'une des revendications 12 à 15, dans lequel l'espace de référence (33) est formé sur la base d'une forme et/ou d'une orientation d'au moins une électrode (31) adjacente à l'espace de réaction (21), l'électrode (31) étant de préférence en forme de cordon et présentant une déviation (32) et/ou un arc pour former l'espace de référence (33).

17. Utilisation d'un dispositif (10) selon l'une des revendications 12 à 16 pour l'usinage de matériau par laser.
